# EUROPEAN PATENT SPECIFICATION

(11) **EP 0 271 227 B1**
(45) Date of publication and mention of the grant of the patent: **09.06.1993**
(21) Application number: 87310006.9
(22) Date of filing: 12.11.1987
(51) Int. Cl.: C12N 15/66, A61K 39/395, C12N 9/72, C07K 15/00, G01N 33/66

(54) **Recombinant hybrid immunoglobulin molecules and their use**
Rekombinante Hybrid-Immunoglobulinmoleküle sowie deren Verwendung
Molécules d'immunoglobulines hybrides et recombinantes ainsi que leur utilisation

(30) Priority: 12.11.1986 US 929581
(43) Date of publication of application: 15.06.1988
(73) Proprietor: THE GENERAL HOSPITAL CORPORATION, Boston, MA 02114 (US)
(72) Inventor: Haber, Edgar, Weston, MA 02193 (US); Quertermous, Thomas, Boston, MA 02115 (US)
(74) Representative: Sheard, Andrew Gregory

(56) References cited:
- EP-A- 0 125 023
- EP-A- 0 146 050
- WO-A-86/01533
- WO-A-87/05934
- SCIENCE, vol. 229, 23 August 1985; C.BODE et al., pp. 765-767*
- JOURNAL OF CELLULAR BIOCHEMISTRY, vol. 32, no. 3, 1986, Alan R. Liss, Inc.; A.-J.VAN ZONNEVELD et al., pp. 169-178*
- PROCEEDINGS OF THE NATL. ACADEMY OF SCIENCES USA, vol. 81, August 1984, P.VERDE et al., pp. 4727-4731*
- SCIENCE, vol. 229, 20 September 1985; S.L.MORRISON, pp. 1202-1207*
- SCIENCE, vol. 222, 09 December 1983; K.Y.HUI et al., pp. 1129-1131*
- GENE, vol. 43, 1986, Elsevier Science Publishers B.V.; G.T.WILLIAMS et al., pp. 319-324*
- NATURE, vol. 312, 13 December 1984; M.S.NEUBERGER et al., pp. 604-608*

## Description

This invention relates to a recombinant hybrid immunoglobulin molecule having an antigen binding site specific for fibrin linked to a second protein comprising the active portion of a plasminogen activator. This invention also is directed to the cloning and production of these novel hybrid immunoglobulin molecules. This invention further relates to a method of using these hybrid immunoglobulin molecules in immunodiagnostic and immunotherapeutic processes.

Most myocardial infarctions are caused by coronary thrombosis (DeWood et al., N. Eng. J. Med., 303:897 (1983)). The coronary thrombosis that causes the myocardial infarction can be lysed by thrombolytic agents. These thrombolytic agents are plasminogen activators that activate the conversion of plasminogen to the fibrinolytic enzyme plasmin. Plasmin will then lyse the fibrin present in the thrombus. This treatment with plasminogen activators is not without side effects. Plasmin acts non-selectively and therefore, not only lyses the fibrin in the thrombus, but also attacks fibrinogen and clotting factors, often resulting in severe bleeding diathesis.

Streptokinase, urokinase, prourokinase, and tissue-type plasminogen activator (tPA) are known plasminogen activators for lysing thrombi. These activators are indicated for the treatment for acute cardiovascular disease such as infarct, stroke, pulmonary embolism, deep vein thrombosis, peripheral arterial occlusion, and other venous thrombosis. Both streptokinase and urokinase, however, have severe limitations. Due to a low affinity for fibrin, both activators will activate circulating and fibrin-bound plasminogen indiscriminately. The plasmin formed in circulating blood is neutralized before it can be used in thrombolysis. Residual plasmin will degrade several clotting factor proteins, for example, fibrinogen, factor V, and factor VIII, causing hemorrhagic potential. Further, streptokinase is strongly antigenic and patients with high antibody titers respond inefficiently to treatment and cannot remain on continuous treatment.

Human tissue-type plasminogen activator can bind to fibrin and therefore favors the activation of plasminogen in close proximity to the thrombus, potentially sparing fibrinogen elsewhere in the circulation. However, at doses required for prompt lysis of coronary thrombi, the use of tissue-type plasminogen activator can also result in hemorrhage.

In order to increase the specificity of the thrombolytic agents to the thrombus, it has been shown that covalent linkage of urokinase to a fibrin-specific antibody results in marked enhancement of fibrinolytic potency and specificity. Bode et al., Science, 229:765-767 (1985).

One function characteristic of every antibody molecule is specific binding to an antigenic determinant. Antibodies in vivo are bivalent and monospecific, containing two identical antigen binding sites. The specific binding of antigen by an antibody molecule is determined by the antibody's structure of the variable regions (F_{ab}) of both heavy and light chains.

Antibodies having dual specificities have been prepared by subjecting antibodies of different specificities to a selective cleavage of the disulfide bridges that link the two heavy chains together. Antibody half-molecules are then reassociated under neutral pH to produce the hybrid antibodies having dual specificities. See Nisonhoff et al., Nature (London), 194:355 (1962); Brennan et al., Science, 229:31 (1985); Liu et al., Proc. Nat'l. Acad. Sci. USA, 82:8648 (1985); and commonly assigned co-pending United States Patent Application, Serial No. 851,554, filed April 14, 1986 (corresponding to published EP-A-0241907).

Bispecific antibodies have also been produced from hybridomas. The preparation of bispecific monoclonal antibodies by fusion of antibody-producing hybridoma cells is described in Milstein and Cuello, Nature (London), 305:537 (1983) and in PCT application, W083 103679.

Antibodies have also been cloned and produced by recombinant DNA techniques. Genes for heavy and light chains have been introduced into appropriate hosts and expressed, followed by reaggregation of these individual chains into functional antibody molecules (see for example Munro, Nature, 312:597 (1984); Morrison, S.L. Science 229:1202 (1985); Oi et al., BioTechniques 4:214 (1986)); Wood et al., Nature, 314U:446-449 (1985)). Light and heavy chain variable regions have been cloned and expressed in foreign hosts, and maintain their binding ability (Moore et al., European Patent Publication 0088994 (published September 21, 1983)).

Chimeric or hybrid antibodies have also been prepared by recombinant DNA techniques. Oi and Morrison, BioTechniques, 4:214 (1986) describes a strategy for producing chimeric antibodies. On pages 218-220 a chimeric:human IgG anti-Leu3 antibody is described. The authors state that a chimeric mouse:human anti-dansyl antibody has been made. This article indicates, without specifically stating, that the Leu3 binding specificity and the anti-dansyl binding specificity have been cloned together into a single immunoglobulin molecule.

Morrison, Science, 229:1202 (1985), in Table 1, states that variable light or variable heavy chain regions can be attached to a non-Ig sequence to create fusion proteins. This article states that the potential uses for the fusion proteins are three: (1) to attach antibody specificity to enzymes for use in assays; (2) to isolate non-Ig proteins by antigen columns; and (3) to specifically deliver toxic agents. There is no description in this reference as to any specific chimeric immunoglobulin molecule.

Neuberger et al., Nature, 314:268 (1985) describes a chimeric antibody whose heavy chain is a human constant region fused to a mouse variable region that is specific for the hapten, 4-hydroxy-3-nitrophenyl-acetyl.

European Patent Application 120,694 describes the genetic engineering of the variable and constant regions of an immunoglobulin molecule that is expressed in E. coli host cells. On page 10 of the application, it states that the immunoglobulin molecule may be synthesized by a host cell with another peptide moiety attached to one of the constant domains. This peptide moiety is described as either cytotoxic or enzymatic. It also states on page 10 that the immunoglobulin molecule may also comprise a therapeutic agent. The description in the application and in the examples describe the use of a lamda-like chain derived from a monoclonal antibody which binds to 4-hydroxy-3-nitrophenylacetal (NP) haptens.

European Patent Application 125,023 relates to the use of recombinant DNA techniques to produce immunoglobulin molecules that are chimeric or otherwise modified. One of the uses described in pages 3-4 for these immunoglobulin molecules is the use of whole body diagnosis and treatment by injecting the antibodies, directed to specific target disease tissues, into a patient. The presence of the disease can be determined by attaching a suitable label to the antibodies, or the diseased tissue can be attacked by carrying a suitable drug with antibodies. The application describes antibodies engineered to aid the specific delivery of an agent as "altered antibodies."

PCT application W083/03971 relates to a hybrid protein that comprises antibody-enzymatically active toxins.

PCT application W083/01533 described on page 5 chimeric antibodies with the variable region of an immunoglobulin molecule linked to a portion of a second protein which may comprise the active portion of an enzyme.

Boulianne et al., Nature, 312:643 (1984) constructed an immunoglobulin gene in which the DNA segments that encode mouse variable regions specific for the hapten trinitrophenyl are joined to segments that encode human mu and kappa constant regions. These chimeric genes were expressed as functional TNP-binding chimeric IgM.

Morrison et al., Proc. Nat'l Acad. Sci. USA, 81:6851 (1984) created a chimeric molecule utilizing the heavy chain variable region exons of an anti-phosphoryl choline myeloma protein gene, which were joined to the exons of either human kappa light chain gene. The genes were transfected into mouse myeloma cell lines, generating transformed cells that produce chimeric mouse-human IgG with antigen binding function.

Sharon et al., Nature, 309:604 (1984) fused a gene encoding a mouse heavy chain variable region specific for azophenylarsonate with the mouse kappa light chain constant region gene. This construct resulted in a polypeptide chain that dimerized with the corresponding V_{L}-Kappa polypeptide chain when introduced into the appropriate myeloma cell line. The V_{Hkappa}V_{L}Cₖₐₚₚₐ molecule was bound to the azophenylarsonate hapten.

Neuberger et al., Nature, 312:604 (1984) joined the heavy chain variable region gene of a hapten-specific antibody to a gene specifying the synthesis of micrococcal nuclease, and obtained a hybrid molecule that had both antigen binding and enzymatic activity.

It would be desirable to have a selective plasminogen activator that is characterized by high affinity and specificity for fibrin relative to fibrinogen, and that would effect activation of plasminogen only in the immediate environment of a fibrin-containing thrombus.

This invention relates to a recombinant hybrid immunoglobulin molecule having an antigen binding site specific for fibrin linked to a second protein comprising the active portion of a plasminogen activator. The invention is also directed to the cloning and production of these novel hybrid immunoglobulin molecules. This invention further relates to a method of using these recombinant hybrid immunoglobulin molecules in immunodiagnostic and immunotherapeutic processes.

The invention also comprises genetic sequences coding for the hybrid immunoglobulin molecules, cloning and expression vectors containing such genetic sequences, hosts transformed with such vectors, and methods of production of such hybrid molecules by expression of the underlying genetic sequences in such hosts. The remainder of the description relates to preferred embodiments of the invention, as do the drawings, in which:

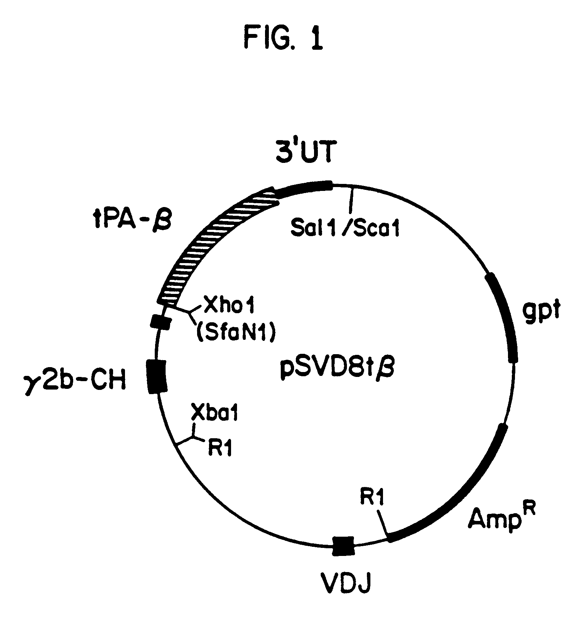

Figure 1 shows the structure of expression plasmid pSVD8t, which codes for the heavy chain-t-PA fusion protein. Coding sequences are indicated by labels outside the circle; restriction sites used in construction are indicated inside the circle. Abbreviations: VDJ, productive 59D8 heavy chain rearrangement; 2b-CH, genomic sequence of the murine 2b heavy chain constant region; t-PA- , cDNA sequence coding for the human t-PA chain; 3'-UT, 3' untranslated sequence of human t-PA cDNA; Amp^{r}, pBR322 ampicillin resistance gene; gpt, E. coli guanine phosphoribosyl transferase gene driven by SV40 promoter; R1, Eco R1.

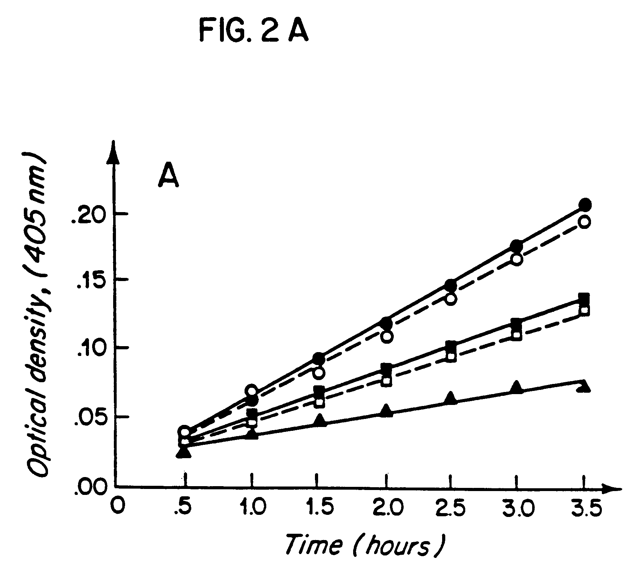

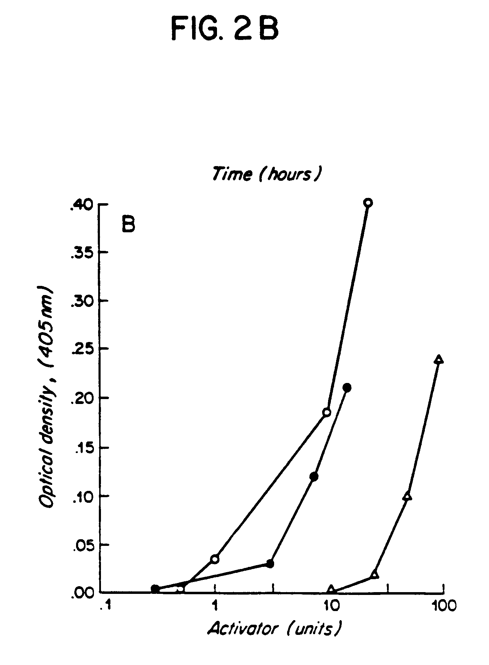

Figure 2 shows the Chromogenic substrate assay comparing the catalytic activity of the recombinant protein with that of melanoma t-PA. Figure 2A: As shown by the dashed lines, the S-2288 assay was performed with 105 ng (open circles) or 70 ng (open boxes) of recombinant protein. Solid lines represent the catalytic activity of 50 ng (filled circles), 40 ng (filled boxes), or 30 ng (filled triangles) of melanoma t-PA used as standards. Relative molar activity of the recombinant protein was determined by comparison with t-PA standards with similar rates of catalysis. Figure 2B: The S-2251 assay was conducted with varying activities of melanoma t-PA standard (open circles), recombinant protein (filled circles), and bovine trypsin (open triangles). Units of activity of each protein were determined in the S-2288 assay.

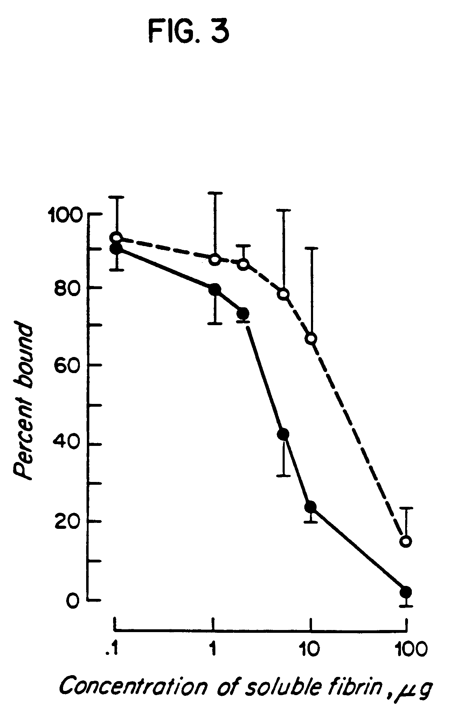

Figure 3 gives a Comparison of the binding behavior of 59D8 antifibrin antibody and recombinant protein. Curves represent the inhibition of antibody binding to solid-phase fibrin monomer by competition with various concentrations of solubles fibrin monomer. Recombinant antibody (dashed lines) requires a slightly higher concentration of soluble fibrin for 50% inhibition than does the antibody (solid lines) and thus binds fibrin somewhat less avidly. This difference is less than 10-fold.

This invention is directed to a hybrid immunoglobulin molecule that has both antigen binding and enzym activity. More specifically, this invention is directed to a recombinant hybrid immunoglobulin molecule having an antigen binding site specific for fibrin linked to a second protein comprising the active portion of plasminogen activator. This invention is also directed to the cloning and production of these novel hybrid immunoglobulin molecules.

Throughout this specification, the term "hybrid immunoglobulin molecule" is used to designate a single molecule produced by recombinant DNA techniques that comprises all, or a portion of, a fibrin-specific antibody and all, or a portion of, a plaminogen activator. Heterobifunctional antibody, heteroantibody, bispecific antibody, heteroligating antibody, antibody duplex, and heterodimer are all terms that refer more specifically to an antibody of dual specificity, that is, two antibody combining sites in one molecule.

Fibrin specificity as used herein refers to antibodies raised against fibrin. When blood escapes from the vasculature, an intricate cascade of enzymatic reactions converts fibrinogen to fibrin, the structural protein in clotted blood. Fibrinogen itself is the least soluble of the plasma proteins. With a 340,000 kd MW, it possesses a two-fold symmetry arising from three pairs of nonidentical polypeptide chains called A-alpha, B-beta, and gamma. At the site of thrombosis, the coagulation cascade is activated to generate thrombin, which enzymatically cleaves polar peptides (Fibrinopeptide A from A-alpha and Fibrinopeptide B from B-beta), and results in fibrin monomer formation. Fibrin monomers, being much less soluble, spontaneously polymerize into a gel network. After polymerization, the fibrin clot is stabilized by Factor XIIIa, which introduces covalent interchain e-(g-glutamyl)lysine bonds. Fibrinogen and fibrin are identical in greater than 98% of their structure and differ only in two newly exposed amino termini, those of the fibrin alpha and beta chains. The amino acid sequence of these fibrin amino termini is known. Doolittle, R.F., "Fibrinogen and Fibrin," in Putnam, F.W., ed. The Plasma Proteins: Structure, Function, and Genetic Control, 3d ed., Vol. 2, New York: Academic Press, 1975; 109-156.

Fibrin epitopes that may be used in this invention include the amino terminus of the fibrin beta chain, the amino terminus of the fibrin alpha chain, the beta (43-49) amino acid sequences, which are carboxy-terminal to a plasmin cleavage site, and the gamma chain crosslink site.

Antibodies with specificity to fibrin have been described in Hui et al., Science, 222: 1129 (1983). Further description of the same type of antibodies can be found in commonly assigned co-pending United States application, Serial No. 824,228, filed January 30, 1986, (US-A-4927916) for "Fibrin-Specific Monoclonal Antibodies Lacking Fibrinogen Cross-Reactivity." Fibrin-specific monoclonal antibodies with substantially no fibrinogen cross-reactivity are also described in commonly assigned co-pending United States Patent Application Serial No. 851,514, filed April 14, 1986. Other examples of antibodies with a specificity against fibrin include Kudryk et al., Mol. Imm., 21: 89 (1984); European Patent Application 146,050 to Callewaert, published June 26, 1985, for "Site Selective Plasminogen Activator and Method of Making and Using Same;" and Australian Patent Application, AU-A-25387/84 to Bundesen et al., for "Monoclonal Antibodies with Specificity for Crosslinked Fibrin and Their Diagnostic Uses."

In preparing the hybrid immunoglobulin molecules of this invention, the entire fibrin-specific antibody may be cloned and comprise a portion of the hybrid molecule. However, in order to reduce the size of the hybrid immunoglobulin molecule, and to reduce antigenicity, it is preferred to use only that region of the antibody that will recognize and bind to fibrin.

Cloning this region of the fibrin-specific antibody requires an understanding of the structure and function of antibodies. Briefly, antibodies are tetrameric immunoglobulins consisting of two identical light (L) chains and two identical heavy (H) chains. Each protein chain consists of two principle regions: the N-terminal variable (V) region and the C-terminal constant (C) region. The variable light (V_{L}) and heavy (V_{H}) chains form the variable region domain. The variable domain determines recognition and specificity to a particular antigen. The constant region domains of light (C_{L}) and heavy (C_{H}) chains mediate the effector function responsible for executing the immune response. The hinge region (J) of the antibody molecule connects the Fab fragment to the Fc fragment of the antibody.

Within the variable region, there may be hypervariable regions known as diversity domains (D). These diversity domains are related to exons observed in the genes encoding for the variable regions.

The variable domain of an antibody, a protein structure definition, consists of both VL and VH segments of the light and heavy chains. It contains 6 hypervariable regions, three in the light chain and three in the heavy chain. On a genetic level, three exons are responsible for specifying VH, including its framework and hypervariable regions; two exons specify VL. The first two hypervariable regions of both VL and VH are specified by the V gene exons of the light and heavy chains respectively. The third hypervariable region of the light chain is specified by two exons, VL and JL. The third hypervariable region of the heavy chain is specified by three exons VH, D, and JH.

Immunoglobulin gene expression occurs through the joining of the V gene to the C gene by somatic recombination in the B lymphocytes. These genes are joined to form the complete immunoglobulin. The rearranged, joined gene segments then encode the complete immunoglobulin or antigen binding domains of light and heavy variable chains.

There are five principal classes of heavy chains, characterized by chemical and isotypic properties. The heavy chain classes are referred to as mu, gamma, delta, alpha, and epsilon, There are also two principal classes of light chains: kappa and lambda.

In this invention, an antibody specific for fibrin is cloned as part of the hybrid immunoglobulin molecule. Preferably, only the variable region of the fibrin antibody is cloned. Either the variable light or variable heavy chain, or both, comprises part of the hybrid molecule. In addition, the hinge region of the fibrin-specific antibody may be cloned. The constant domain of the Fab portion of the fibrin-specific antibody joined to the variable region may also be cloned.

The variable and constant region of the fibrin-specific antibody cloned and used in the hybrid immunoglobulin molecule may be derived from a mammalian source, with the preferred source from humans. Alternatively, the variable region may be from a mammalian source, with the constant region from a human source.

Plasminogen activators that may be used in this invention include urokinase, prourokinase, tissue type plasminogen activator, and streptokinase. When plasminogen is converted by an activator to plasmin, the active fibrinolytic enzyme of plasma, it develops a marked affinity for its substrate, fibrin.

The term "plasminogen activator" is therefore a thrombolytic agent and is meant to include in this specification any agent utilized. Other terms are known in the art for the lysis of a thrombus, including fibrinolysis. Although the most common plasminogen activators are streptokinase, urokinase, prourokinase, and tissue-type plasminogen activator, any other plasminogen activator or thrombolytic agent may be used in this invention.

In preparing the hybrid immunoglobulin molecules of this invention, the entire plasminogen activator may be cloned and expressed as part of the hybrid molecule. Preferably, only the active portion of the plasminogen activator is cloned. This active site or catalytic site may be determined by routine screening as described in the examples.

The process for obtaining a hybrid immunoglobulin molecule according to the present invention requires the cloning of the fibrin-specific antibody and the plasminogen activator and expression of their DNA sequences into a single hybrid molecule.

The DNA sequences of the fibrin-specific antibody and the plasminogen activator employed for preparation of the hybrid immunoglobulin molecule may be derived from a variety of sources. These sources include genomic DNA, cDNA, synthetic DNA, and combinations thereof. The genomic DNA may or may not include naturally occurring introns.

The DNA obtained from the genomic DNA or cDNA may be obtained in a variety of ways. Cells coding for the desired sequence may be isolated, the genomic DNA fragmented, conveniently by one or more restriction endonucleases, and the resulting fragments cloned and screened with a probe for the presence of the DNA sequence coding for fibrin-specificity or for the plasminogen activator.

For the variable region of the fibrin-specific antibody, the rearranged heavy chain coding DNA may include V, D, and J regions. The rearranged germline light chain coding DNA may include the V and J regions. Once the cloned fragment has been identified which contains the desired fibrin-specific DNA sequence binding site, this fragment may be further manipulated to remove superfluous DNA, modify one or both termini, remove all or a portion of intervening sequences, (introns) or the like.

The joining of the various fragments is performed in accordance with conventional techniques, employing blunt-ended or staggered-ended termini for ligation, restriction enzyme digestion to provide for appropriate termini, filling in of cohesive ends as appropriate, alkaline phosphatase treatment to avoid undesirable joining, and ligation with appropriate ligases.

For cDNA, the cDNA may be cloned and the resulting clone screened with an appropriate probe for cDNA coding for the desired variable or constant region. Once the desired clone has been isolated, the cDNA may be manipulated in substantially the same manner as the genomic DNA. However, with cDNA there will be no introns or intervening sequences.

Further, the genes of the fibrin-specific antibody and the genes of the plasminogen activator may be synthesized according to well-known means and cloned for use in preparing the hybrid immunoglobulin molecule.

To express the hybrid immunoglobulin molecule, transcriptional and translational signals recognized by an appropriate host are necessary. Eukaryotic hosts will be mammalian cells capable of culture in vitro, particularly leukocytes, more particularly myeloma cells, or other transformed or oncogenic lymphocyte, e.g., EBV transformed cells. Alternatively, non-mammalian cells may be employed, such as bacteria, fungi, e.g., yeast, filamentous fungi, or the like.

The DNA sequence coding for the fibrin-specific variable region may be obtained in association with the promoter region from genomic DNA. To the extent that the host cells recognize the transcriptional regulatory and translational initiation signals associated with the variable region, then the region 5' of the variable region coding sequence may be retained and employed for transcriptional and translational initiation regulation.

The contiguous non-coding region 5' to the variable region will normally include those sequences involved with initiation of transcription and translation, such as the TATA box, capping sequence, CAAT sequence, and the like. Usually the 5'-non-coding sequence will be at least 150bp, more usually at least 200bp, usually not exceeding about 2kbp, more usually not exceeding about 1kbp.

The non-coding region 3' to the fibrin specific constant region may be retained for its transcriptional termination regulatory sequences, such as termination and polyadenylation. In addition, the non-coding region 3' to the coding region also contains an important enhancer in immunoglobulin genes. Thus, by retaining the 3'-region naturally contiguous to the DNA sequence coding for the constant region, the transcriptional termination signals may be provided. Where the transcriptional termination signals are not satisfactorily functional in the expression host cell, then a 3' region functional in the host cell may be substituted.

The constructs for the fibrin-specific antibody and the plasminogen activator may be joined together to form a single DNA segment or may be maintained as separate segments, by themselves or in conjunction with vectors.

The construct(s) may be introduced into a cell by transformation in conjunction with a gene allowing for selection where the construct will become integrated into the host genome. Usually the construct will be part of a vector having a replication system recognized by the host cell.

Expression vehicles for production of the molecules of the invention include plasmids or other vectors. In general, such vectors containing replicon and control sequences which are derived from species compatible with a host cell are used in connection with the host. The vector ordinarily carries a replicon site, as well as specific genes which are capable of providing phenotypic selection in transformed cells. For example, E. coli is readily transformed using pBR322, a plasmid derived from an E. coli species. pBR322 contains genes for ampicillin and tetracycline resistance, and thus provides easy means for identifying transformed cells. The pBR322 plasmid or other microbial plasmids must also contain, or be modified to contain, promoters which can be used by the microbial organism for expression of its own proteins. Those promoters most commonly used in recombinant DNA construction include the beta lactamase, lactose promoter systems, lambda phage promoters, and the tryptophan promoter systems. While these are the most commonly used, other microbial promoters have been discovered and can be utilized.

For example, a genetic construct for the hybrid immunoglobulin molecule can be placed under the control of the leftward promoter of bacteriophage lambda. Control is exerted by the lambda repressor, and adjacent restriction sites are known.

The expression of the hybrid immunoglobulin molecule can also be placed under control of other regulatory sequences which may be homologous to the organism in its untransformed state. For example, lactose dependent E. coli chromosomal DNA comprises a lactose or lac operon which mediates lactose utilization by elaborating the enzyme beta-galactosidase. The lac control elements may be obtained from bacteriophage lambda plac5, which is infective for E. coli. The lac promoter-operator system can be induced by IPTG.

Other promoter/operator systems or portions thereof can be employed as well. For example, colicin E1, galactose, alkaline phosphatase, tryptophan, xylose, tax, and the like can be used.

The preferred hosts are mammalian cells, grown in vitro in tissue culture, or in vivo in animals. Mammalian cells provide post translational modifications to immunoglobulin protein molecules including correct folding or glycosylation at correct sites.

Mammalian cells which may be useful as hosts include cells of fibroblast origin such as VERO or CHO-K1, or cells of lymphoid origin, such as the hybridoma SP2/0-AG14 or the myeloma P3x63Sg8, and their derivatives. Preferred mammalian host cells include SP2/0 and J558L. Several cell lines secrete urokinase and may be used for transfection, such as cultured kidney carcinoma cells (Ferraivolo, et al., J. Cell. Physiol., 121:363 (1984)) and 3T3 cells (Belin, et al., EMBO J., 3:190 (1984)).

For a mammalian host, several possible vector systems are available for the expression of the hybrid immunoglobulin molecule. One class of vectors utilizes DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotropic host, biocide resistance, e.g. antibiotics, or heavy metals, such as copper, or the like. The selectable marker gene can either be directly linked to the DNA gene sequences to be expressed, or introduced into the same cell by co-transfusion, Additional elements may also be needed for optimal synthesis of single chain binding protein mRNA. These elements may include splice signals, as well as transcription promoters, enhancers, and termination signals. cDNA expression vectors incorporating such elements include those described by Okayama, H., Mol. Cel. Biol., 3:280 (1983), and others.

A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

Another preferred host is yeast. Yeast provides substantial advantages in that it can also carry out post translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products, and secretes peptides bearing leader sequences (i.e., pre-peptides).

Any of a series of yeast gene expression systems incorporating promoter and termination elements from the actively expressed genes coding for glycolytic enzymes produced in large quantities when yeast are grown in mediums rich in glucose can be utilized. Known glycolytic genes can also provide very efficient transcription control signals. For example, the promoter and terminator signals of the phosphoglycerate kinase gene can be utilized.

Once the vector or DNA sequence containing the construct(s) has been prepared for expression, the DNA construct(s) may be introduced into an appropriate host. Various techniques may be employed, such as protoplast fusion, calcium phosphate-precipitation, electroporation or other conventional technique. After the fusion, the cells are grown in a selective medium, where untransformed cells are killed, leaving only cells transformed with the DNA construct. Expression of the gene(s) results in assembly to form the hybrid immunoglobulin molecule.

The host cells will for the most part be immortalized cells, particularly myeloma or lymphoma cells. These cells may be grown in an appropriate nutrient medium in culture flasks or injected into a synergenic host, e.g., mouse or rat, or immunodeficient host or host site, e.g., nude mouse or hamster pouch. Particularly, the cells may be introduced into the abdominal cavity for production of ascites fluid and harvesting of the chimeric receptor. Alternatively, the cells may be injected subcutaneously and the antibodies harvested from the blood of the host. The cells may be used in the same manner as the hybridoma cells. See Diamond et al., N. Eng. J. Med. (1981) 304:1344 and Kennatt, McKearn and Bechtol (eds.), Monoclonal Antibodies: Hybridomas--A New Dimension in Biological Analysis, Plenum, 1980, which are incorporated herein by reference.

The hybrid immunoglobulin molecule may be isolated and purified in accordance with conventional conditions, such as extraction, precipitation, chromatography, affinity chromatography, electrophoresis, or the like. The preferred method is affinity chromatography with either the amino terminal heptapeptide of the fibrin beta chain (binds to the antifibrin site) or with benzamidine (binds to the plasminogen activator catalytic site) to selectively isolate the hybrid molecule.

The present invention also provides methods for immunotherapy and immunodiagnosis using the hybrid immunolglobulin molecules. In the immunotherapeutic and immunodiagnostic applications, the hybrid immunoglobulin molecule is administered to a patient, which becomes localized at the site of a thrombus through the fibrin-specific binding site of the hybrid molecule. The thrombus is lysed by the enzyme activity of the plasminogen activator portion of the hybrid molecule. As will be appreciated by one of skill in the art, the specificity of the fibrin specific-plasminogen activator hybrid molecule permits selectivity of attachment to and lysis of the thrombus which reduces the risk of serious side effects, such as hemorrhage.

The hybrid immunoglobulin molecules of this invention may also be used in immunodiagnostic applications, including in vivo immunodiagnosis. In this application, the hybrid molecule is detectably labelled using a radionuclide. The radionuclide must be of the type of decay which is detectable for a given type of instrument. Further, the radionuclide for in vivo diagnosis should have a half-life long enough that it is still detectable at the time of maximum uptake, but short enough that after diagnosis unwanted radiation does not remain in the patient. Coupling of the radionuclides to the protein and thus to the hybrid molecule, is known in the art and is often accomplished either directly or indirectly using an intermediary functional group. Examples of radioisotopes that can be used for in vivo diagnosis are ⁹⁹Tc, ¹²³I, ¹³¹I, ¹¹¹In, ⁹⁷Tu, ⁶⁷Cu, ⁶⁷Ga, ⁶⁸Ga, ⁷²As, ⁸⁹Zr, and ²⁰¹T1.

Paramagnetic isotopes for purposes of in vivo diagnosis can also be used according to the methods of this invention. Examples of elements that are particularly useful for use in Magnetic Resonance Energy techniques include ¹⁵⁷Gd, ⁵⁵Mn, ¹⁶²Dy, ⁵²Cr, and ⁵⁶Fe.

The hybrid immunoglobulin molecule can further comprise a pharmaceutical composition, with a pharmaceutically acceptable carrier. These carriers are well known in the art and can include aqueous or solvent emulsions or suspensions, including saline and buffered media. The pharmaceutical art, for example, as described in Remington's Pharmaceutical Sciences (16th Edition, 1980).

The dose ranges for administration of the hybrid immunoglobulin molecule are those that are large enough to detect the presence of thrombi. The dosage should not be so large as to cause adverse side effects, such as hypersensitivity reactions such as rashes or anaphylactic shock. Generally, the dosage will vary with the age, conditions, sex, and extent of disease in the patient. Counter indications can include hypersensitity and other variables and can be adjusted by the individual physician. Dosage can range from 0.01 mg/kg to 500 mg/kg of body weight, preferably 0.01 mg/kg to 200 mg/kg. The hybrid immunoglobulin molecule(s) can be administered parentally by injection or by gradual perfusion over time. They can also be administered intravenously, intraperitoneally, intramuscularly, or subcutaneously.

Having now generally described this invention, the same will become more readily understood by reference to specific examples included herein for purposes of illustration only, and are not intended to be limiting unless otherwise specified.

### EXAMPLES

### EXAMPLE 1

### Materials.

N-succinimidyl 3-(2-pyridyldithio) propionate (SPDP) and 2-iminothiolane were obtained from Pierce, and Sepharose 4B-CL was obtained from Pharmacia. The ¹²⁵I fibrinogen (IBRIN) came from Amersham. Plasma was purchased from the local blood bank. A chromogenic substrate for porteases, H-D-isoleucyl-L-prolyl-L-arginine-p-nitroanalide dihydrochloride (S-2288) was obtained from Helena Labs. All other chemicals came from either Sigma or Fisher.

Fibrin-specific antibody 64C5 has been described in Hui et al., supra, and in commonly owned and co-pending United States Patent Application, Serial No. 824,228, incoporated herein by reference. Fibrin-specific antibody 59D8 has been described in commonly owned and co-pending United States Patent Application, Serial No. 851,514, incorporated herein by reference.

### Electrophoresis and autoradiography.

SDS-PAGE was performed according to the method of Laemmli, Nature (London), 277:681 (1970). Proteins were visualized using either Coomassie Brilliant Blue R or, where radiolabeled, by autoradiography for 24-72 hours at -70°C.

### Cloning the urokinase gene.

A complementary DNA clone (PHUK8) containing the catalytic B chain coding sequence has been obtained as a gift from Dr. F. Blasi (Verde et al., Proc. Nat'l. Acad. Sci. USA, 81:4727 (1984)). This clone has been grown and isolated in quantity in pBR322. A segment of this DNA clone is used in constructs with antifibrin antibody genes, and it is used to isolate the genomic DNA sequence.

### Cloning the tPA gene.

A full length complementary DNA clone of the human tPA gene (PPA34'F) has been supplied as a gift by Dr. Sandra Degen. The sequence coding for the catalytic B chain of tPA has been extracted from the clone and a unique short segment of synthetic DNA (adapter) coding for two common restriction enzyme cleaveage sites placed at the 5' end of this sequence. This adapter allows the gene to be conveniently joined "in-frame" to other segments of DNA. This tailored clone is used in constructs with antifibrin antibody genes. It is also used to isolate the genomic sequence coding for the tPA B chain, and the genomic sequence subsequently used in constructs with the 59D8 genes.

### Cloning the variable light and heavy chain genes of the fibrin-specific antibody 64C5.

Using a redundant oligonucleotide 17-mer, which was synthesized to correspond to the amino terminus for the light-chain of 64C5 and an available kappa/J probe, the procedure 64C5 light chain rearrangement was cloned from a subgenomic library constructed in pBR322. The entire light chain was reconstructed by ligating the cloned rearranged fragment into a pBR322 plasmid that contains a 5.8Kb EcoR1/BamH1 fragment which encodes the mouse kappa constant region and joining segments (Max, et al., J. Biol. Chem., 256:5116-20 (1981)). The VJ rearrangement was placed 5' of the constant region in the correct orientation. Genomic DNA derived from the 64C5 hybridoma was digested with EcoR1 restriction enzyme, size fractionated on a preparative agarose gel, and subsequently ligated into a lambda coning vector to produce a subgenomic library. Using a probe from the heavy chain joining region, a rearranged fragment was cloned. It was determined to be the heavy chain rearrangement used in the 64C5 hybridoma by hybridization to an oligonucleotide based on the amino terminal sequence of the antibody.

### Cloning the variable heavy chain gene of the fibrin-specific antibody 59D8.

Genomic DNA from the 59D8 hybridoma was size fractionated, ligated into a lambda cloning vector and screened with the heavy chain joining region probe noted above. Both heavy chain rearrangements were cloned and the productive one was selected by hybridization to an oligonucleotide 20-mer based on RNA seuqence of the 59D8 heavy chain.

### Fibrin-specific antibody/plasminogen activator genetic constructs.

The cloned restriction fragment, containing variable and joining region as well as enhancer sequences of the 59D8 or 64C5 gene, is inserted in correct orientation into a plasmid 5' of the mouse gamma 2B heavy chain constant region sequence. This plasmid containing the constant region sequence (PSV GPT/gamma 2B) also contains the ampicillin resistance gene from pBR322, and the guanine phosphoribosyl transferase (GPT) gene under control of the SV40 viral promoter. It was obtained as a gift from Dr. Richard Neer. This construct is propagated in E. coli MC1061 via the ampicillin resistance gene, and expression of the GPT gene in eukaryotes could be selected for in the presence of xanthine, hypoxanthine, and mycophenolic acid. The bulk of the sequence coding for the carboxy terminus of the heavy chain constant region was subsequently removed. It has been replaced with a complementary DNA fragment which codes for the catalytic carboxy "B" chain or urokinase or tissue plasminogen activator. The third exon from either one of the heavy chain constant region genes is joined "in frame" to one of the plasminogen activator genes such that the usual amino acid sequence will be produced, and a composite protein will result, This final construct is transfected via electroporation into the appropriate 59D8 or 64C5 hybridoma variant which has stopped producing the usual heavy chain. These transfectants produce an antibody molecule with fibrin specificity, with a plasminogen activator moiety at the tail end of the truncated heavy chain.

### Purification of urokinase-64C5 hybrid immunoglobulin molecules.

The hybrid molecule is isolated utilizing successive affinity chromatography on benzamidine-Sepharose and beta-peptide-Sepharose in order to obtain hybrid molecules that contain both an antibody combining site and urokinase or tPA sequence. The affinity column is constructed by coupling a synthetic amino terminal beta chain fibrin peptide (Gly-His-Arg-Pro-Leu-Asp-Lys-Cys (beta peptide)) Hui, et al., Science, 222:1129 (1983) to maleimidokenzoyl lysine-Sepharose C1-4B Kitagawa, et al., J. Biochem. (Japan), 79:233 (1976). The eluate of this column (0.2 M glycine HCl, pH2.8) is recovered and analyzed to determine fibrin binding properties and fibrinolytic activity.

### Purification of tPA-59D8 hybrid immunoglobulin molecules.

Hybrid immunoglobulin molecules are purified from the host cells by sequential affinity chromatography in two steps. The molecules are first applied to Sepharose CL-4B containing immobilized benzamidine: tPA-antibody hybrid molecules are retained and later eluted with 0.1 M acetate, 0.4 M NaCl (pH 4.0). After neutralization, the eluate is then applied to the peptide-Sepharose column. The eluate from the beta peptide column (0.2M glycine, pH 2.8), is dialyzed against NaPi buffer including 1.0 M arginine and 0.1% Tween 80 and was stored in this buffer at 4 degrees.

### Characterization of hybrid immunoglobulin molecules.

The hybrid molecules are subjected to SDS-PAGE under both reducing and nonreducing conditions. The gels are either stained with coomassie blue or subjected to autoradiography (if tPA or UK is labeled with ¹²⁵I before coupling).

### Chromogenic substrate assay for peptidase activity.

To assess the functional properties of the hybrid molecule, its peptidolytic properties are first examined with respect to a nonselective substrate, H-D-isoleucyl-L-prolul-L-arginine-p-nitroanalide dihydrochloride (S-2288). The S-2288 assay is performed with a total volume of 1,0 ml in 0.05 M Tris-HC1, 0.10 M NaCl (pH 8.5) with a substrate concentration of 3 x 10 ⁴ M. Absorbance at 405 nm is measured every 10 seconds at 20°C.

### Fibrinogen assays.

The fibrinogen content of samples of citrated human plasma or citrated rabbit plasma was determined by two methods. Clottable fibrinogen was measured by the method of Clauss, Acta Chir. Scand., 90:419 (1957), and total fibrinogen is determined by sodium sulfite precipitation.

### Plasma clot assay.

The method of Lijnen et al., Thromb. Haemostas., 52:308 (1984) is used with the following modifications. Human fresh-frozen plasma obtained from donors is pooled, aliquoted, and refrozen. Immediately before each experiment, the activities of tPA, UK and their hybrid immunoglobulin molecules are calibrated using the S-2288 assay (i.e., the peptidase activities of the native plasminogen activators and their hybrid molecules are determined and appropriate dilutions made so that the peptidase activity [in units/ml] is identical for each sample). Plasma clots are made by adding each of the following to fresh-frozen plasma: thrombin, 8 NIH units/ml; 0.5 M CaCl₂, 100 ul/ml; and ¹²⁵I-labeled human fibrinogen (IBRIN), 40,000 cpm/ml. The solution is immediately drawn into Silastic tubing (I.D. = 4 mm), and incubating at 37°C for 30 min. Silastic tubing containing clotted fresh-frozen plasma is cut into 1.5-cm sections, yielding clots of 0.2 ml. These are then washed in 0.15 M NaCl before use. Each clot is placed in a plastic tube, counted, and suspended in 1 ml fresh-frozen plasma (from the same pool). Experiments are initiated by the addition of a plasminogen activator (or hybrid molecule of plasminogen activator and antibody). At 30 minute intervals, an aliquot of the fresh-frozen plasma is removed from each tube for counting. Samples are saved at the end of the experiment for determination of fibrinogen levels.

### In vivo thrombolysis.

The rabbit jugular vein model of Collen et al., J. Clin. Invest., 71:368 (1983), is used. After sedation of the rabbit with acetopromazine and ketamine, a paramedial incision is made from the right mandible to above the right clavicle. The external jugular vein is isolated by dissection, and branches are ligated and separated. A segment of woolen thread is introduced to anchor the cot. After bleeding ceases, vascular clamps are placed so as to isolate this segment of the external jugular vein, and the components of the clot are introduced into the isolated vein segment. These components consist of approximately 500,000 cpm of ¹²⁵I-labeled human fibrinogen (each sample is counted before use), 100 ul of packed human red blood cells, 100 ul of human fresh-frozen plasma, 10ul of 0.5 M CaCl₂ and 10 ul of bovine thrombin (8 NIH units). After 30 minutes, the vascular clamps are removed and blood flow is restored. A sample of blood is taken immediately after the clamps are released to determine radioactivity not incorporated into the thrombus. Measured amounts of plasminogen activator are diluted to a volume of 25 ml, and are delivered via the marginal vein of the contralateral ear over 4 hours by infusion pump. Lost counts are determined by counting syringes, gauze sponges and tubing. Six hours after initiation of the infusion, the entire vein segment is isolated, removed and counted. Percent lysis is determined as the ratio of the counts remaining at the termination of an experiment over the net counts at the beginning.

### Fibrinolytic assay.

The quantitative fibrinolytic assay links fibrin monomer to Sepharose. Fibrinogen is purified of plasmin contaminates by passage over lysine-Sepharose and then mixed with trace labelled ¹²⁵I-fibrinogen. It is then coupled to cyanogen bromide activated Sepharose C1-4B. The immobilized fibrinogen is converted to fibrin by addition of human thrombin in the presence of 100 mM CaC1₂.

To assess their relative fibrinolytic activity, increasing amounts of ¹²⁵I-UK-64C5 hybrid molecule and urokinase (or ¹²⁵I-tPA-59D8 hybrid molecule and tPA) are incubated with ¹²⁵I-fibrin-Sepharose for 4 hours. Thereafter, the resin is incubated with purified plasminogen. After intervals of 2.5 and 15 hours, the mixture is centrifuged and the radioactivity of the supernatant is determined. This procedure is repeated with the control conjugate, (¹²⁵I-UK)-SS-(3H3).

### EXAMPLE 2

### MATERIALS AND METHODS

### Cloning of 59D8 Heavy Chain Gene.

High molecular weight genomic DNA was made from the 59D8 hybridoma cells as previously described in Quertermous et al., J. Immunol., 128:2687-2690 (1987). To identify rearranged heavy chain immunoglobulin genes specific for the 59D8 hybridoma line, Southern blot analysis was performed as previously described with Eco R1-digested genomic DNA and a 1.7-kilobase (kb) Eco R1/Pst1 genomic joining region probe. (Southern, E.M., J. Mol. Biol., 98:503-517; Sakano et al., Nature, 286:676-683 (1980). Two rearrangements were identified that were not found in either of the cells originally fused to produce the 59D8 hybridoma (SP2/0 and Balb/c). Subsequently, one milligram of genomic DNA was digested with Eco R1 and size-fractionated on a preparative agarose gel. Southern, E. in Methods in Enzymology, et. Wu, R. (Academic Press, NY) vol. 68, pp. 152-176. Fractions containing each of the two rearranged fragments were identified by hybridization to the joining region probe. These fractions were concentrated and ligated into λgt10. The two subgenomic libraries thus constructed were screened with the joining region probe and several potential clones were isolated from each library. (Maniatis et al., Molecular Cloning, 1982 (Cold Spring Harbor, NY). Selection of the clone containing the rearranged fragment coding for the 59D8 antigen combining site was accomplished by hybridization to a 20-basepair oligonucleotide that had been constructed on the basis of the sequence of the 59D8 heavy chain mRNA. RNA isolation and sequencing, ³²P labeling of the oligonucleotide with T4 polynucleotide kinase, and hibridization were carried out according to previously described techniques. (Maniatis et al., Molecular Cloning, supra; Clarke et al., J. Exp. Med., 161:687-704 (1985); Suggs et al., Proc. Nat'l Acad. Sci. USA, 78:6613-6617 (1981).

### Expression Vector Construction

The t-PA sequence was derived from a cDNA clone (pPA34'F) that had been constructed from HELA cell mRNA. Fisher et al., J.Biol. Chem., 260:11223-11230 (1985). DNA encoding the β chain SacI site to the Eco R1 site of pBR322 was isolated and ligated into pGEM3. Next, a contiguous 5' fragment was isolated by digestion with SfaN1 and Sac1. synthetic oligonucleotide, containing a Bam H1 end, an Xho1 site, and two bases reconstituting a codon for glycine, was added to this second fragment's 5' end. The modified fragment was then ligated into a plasmid already containing the 3' fragment, thus reconstituting the β chain sequence. The β chain was excised with Xho1 and Sca1 - the Sca1 site being contributed by the pBR322 sequence.

The final construct was assembled in the pSV2gpt vector that had been modified by the insertion of a polylinker containing a 6-kb Xbal restriction fragment encoding the murine γ2b heavy chain constant region. Mulligan et al., Proc. Nat'l Acad. Sci. USA, 78:2072-2076 (1981); Tucker et al., Science, 206:1303-1306 (1979). The productive 59D8 heavy chain rearranged gene that had been cloned on a 2.6-kb Eco R1 fragment was inserted in the correct orientation into an Eco R1 site in the polylinker 5' of the γ 2b constant region. The constant region sequence between the unique Xho1 site in CH2 and a Sal1 site in the polylinker was excised, the sal1 site was blunted and the t-PA β chain was ligated into place. Nucleotide sequence analysis confirmed that the junction between the heavy chain and t-PA segments was in-frame. Sanger et al., Proc. Nat'l Acad. Sci. USA, 74:5463-5467 (1977).

### Monoclonal Antibodies and Selection of Loss Variants.

Fibrin-specific monoclonal antibody 59D8 was raised by immunization with a synthetic heptapeptide based on the amino terminal sequence of the fibrin β chain, as previously described in Hui et al., Science, 222:1129-1132 (1983). Hybridoma cells and loss variants were maintained in complete medium: DMEM with 4.5 mg/ml glucose, 12 percent fetal calf serum (FCS), 50 g/ml gentamicin sulfate, and 0.6 mg/ml L-glutamine. For selection of heavy chain loss variants, cells were grown in soft agarose. Five milliliters of complete medium plus 0.2 percent agarose and an additional 8 percent FCS was added to tissue culture dishes (60 mm) and allowed to solidify at room temperature for 3 to 5 min. Cells (1 to 2 x 10³) to be selected for chain loss were layered over the agarose. The plates were incubated at 37°C in 6 percent CO₂ until clusters of cells were formed (2 to 4 days). To detect heavy chain loss variants, cell clusters were overlayed with an antiserum solution 1.0 ml) containing complete medium with 0,2 percent agarose and 5 to 10 percent rabbit or goat anti-mouse heavy chain. Cell clusters secreting heavy chain developed a precipitin halo. Clusters that did not have a precipitin halo were picked from soft agarose by capillary pipet and subsequently delivered into 96-well plates containing complete medium with 8 percent additional FCS. Individual sublones were assayed by enzyme-linked immunoabsorbent assayh (ELISA) or by Western blotting for the presence of heavy and light chain.

### Transfection and selection.

The construct pD8SVtβ was transfected into loss variant cells by electroporation, using an Isco power supply as decribed in Potter et al., Proc. NAt'l Acad. Sci. USA, 81:7161-7165 (1984). Optimal transfection conditions were a 2000-volt discharge into 0.8 ml of phosphate buffered saline. Transformants were selected by growth in mycophenolic acid, xanthine and hypoxanthine. Confirmation of transfection and expression was obtained by Northern blot analysis using a 2-kb cDNA probe coding for the 3ʹ portion of the human t-PA β chain. Maniatis et al., Molecular Cloning supra. Transfected cell lines were subcloned according to standard techniques.

### Protein Purification.

Protein was purified from cell supernatants and from ascites by sequential double affinity chromatography on two columns. One column was constructed by linking the synthetic peptide used for the generation of 59D8 to Sepharose. The other consisted of an anti-human t-PA monoclonal antibody linked to Sepharose. We had used a third column, composed of benzamidine linked to Sepharose, in our initial purification attempts. However, even though benzamidine binds well to the active site of t-PA and benzamidine-Sepharose can be used to purify the intact molecule, the column did not retain the recombinant protein.

Purification of the recombinant protein was monitored by two solid-phase immunoassays. To detect antifibrin antibody activity, 96-well microtiter plates were coated with fibrin monomer and blocked with 10 percent horse serum. They were then incubated with samples, and washed and probed with ¹²⁵I-labeled goat anti-mouse Fab. The second assay was designed to detect t-PA antigen associated with antifibrin antibody activity. In this assay, the fibrin-monomer-coated plates were incubated with culture supernatant or ascites and probed with ¹²⁵I-labeled anti-human t-PA. Because the chain of t-PA possesses no fibrin binding activity, only recombinant protein containing both functional domains is detected.

### Western Blot Analysis.

Western blots were made from both reduced and nonreduced samples separated on NaDodSO₄ polyacrylamide gels using established techniques. Burnette, W.N., Anal. Biochem., 112:195-203 (1981). Either goat anti-mouse Fab or a monoclonal anti-human t-PA antibody labeled with ¹²⁵I was used as a probe.

### Antigen Binding Assay.

The original antibody (59D8) and the recombinant molecule were first assayed for the presence of fibrin-binding Fab antigen. This was accomplished with the solid-phase immunoassay described above using ¹²⁵I-labeled goat anti-mouse Fab as a probe. Titration curves were generated for 59D8 and the recombinant protein by varying their concentrations in the assay. That concentration which would yield the same amount of bound ¹²⁵I-labeled antibody was then selected from the linear part of each curve. At this concentration of either 59D8 or fusion protein, a competition assay was performed in wells that had been coated with fibrin and filled with various amounts of soluble fibrin. Protein that bound to the soluble rather than insoluble fibrin was removed by washing before application of the labeled antibody.

### Assays of Enzymatic Function.

To compare the enzymatic function of the recombinant protein with that of native t-PA, its peptidolytic properties were first examined in an assay which measures cleavage of the nonselective substrate S-2288 (Helena Labs, Beaumont, TX). The assay was carried out in a 50 µl volume of buffer (0.15 M Tris, 015 M NaCl) with a 1 millimolar final concentration of chromogenic substrate. Various concentrations of recombinant protein or t-PA purified from the Bowes melanoma cell line (Bio Response, Hayward, CA) were added and the absorbance at 405 nm was measured at a series of time points.

To determine whether the recombinant protein was capable of activating plasminogen, a second assay was performed utilizing the chromogenic substrate S-2251 (Helena Labs). The activity of melanoma t-PA , the recombinant protein and bovine trypsin were first determined in the S-2288 assay and the concentrations were adjusted such that each enzyme was present at 100 units/100 µl. One hundred µl of melanoma t-PA, recombinant protein or bovine trypsin was then added in serial dilution to 100 µl of human plasminogen (0.15 mg/ml), and 800 µl of S-2251 substrate. The samples were incubated for 60 min at 37° C. The reaction was terminated by the additional of 1 ml of 50 percent acetic acid and absorbance at 405 nm was determined.

### RESULTS

Electroporation of the construct pSVD8T (Fig. 1) into the 59D8 heavy chain loss variants provided numerous transfected clones. When approximately 1 x 10⁸ hybridoma cells were mixed with 50 µg of circular plasmid DNA in 0.8 ml of phosphate buffered saline and subjected to a discharge of 2000 volts, approximately 15 of the wells on a 96-well plate contained drug-resistant clones. Approximately 75 percent of these clones, were shown to secrete the recombinant protein. Five clones were chosen for further analysis on the basis of their growth rate and expression of mRNA coding for the fusion protein.

Westerm blot analysis of the affinity-purified recombinant protein were done. Blots of reduced gels probed with an iodinated anti-human t-PA monoclonal antibody revealed labeling of a 65-kD peptide. This is the expected size of a heavy chain-t-PA fusion protein. The β chain of t-PA is approximately 33 kD and the truncated heavy chain should contribute 30 kD. Several lines of evidence indicate that the 65 kD peptide is not a t-PA-like molecule contributed by fetal calf serum. The 65-kD band is observed when the transfected cell lines are grown in serum-free medium or in the intraperitoneal space of mice. Also, when we purified bovine t-PA from fetal calf serum by benzamidine affinity chromatography, even though it was labeled by the antibody on Western blots, the size of the molecule was 75 kD.

Wester blots of reduced samples probed with a goat anti-mouse Fab derived from polyclonal sera reveal labeling of a 25-kD protein, which is the expected size of the 59D8 κ light chain. Although on such blots this reagent usually labels the mouse immunoglobulin heavy chains also, the absence of labeling of the fusion peptide is not surprising since most of heavy chain constant region has been removed. Blots produced with unreduced samples show labeling of a single band at a molecular weight of 170-180 kD by both of the iodinated antibodies. This provides strong evidence that the hybridoma cells are producing a molecule containing both immunoglobulin and t-pa peptides. The 170-180 kD value suggests that the inter-heavy-chain disulfide bonds have formed to give a Fab'₂-like molecule that contains two antigen combining sites and two t-PA moieties.

The peptidolytic activity of the t-PA portion of the molecule was initially assessed by measuring the cleavage of the nonspecific substrate S-2288. Cleavage of this tripeptide can be accurately monitored by following the production of paranitroaniline, which absorbs light at a wavelength of 405 nm. Fig. 2A shows a typical assay, which employs directly the activity of differing concentrations of pure melanoma t-PA. Activity in this assay is defined as the rate of change in optical density. When a comparison is made on a molar basis between the recombinant protein and native t-PA, the recombinant protein possesses 70 percent of the activity of native t-PA.

To determine whether the catalytic β subunit maintained activity against plasminogen (its physiologic substrate), an S-2251 assay was performed. Here the plasminogen activator is required to convert plasminogen to plasmin and the plasmin subsequently liberates paranitroaniline from a synthetic tripeptide. Neither plasminogen activator nor trypsin can directly convert the S-2251 substrate. The amidolytic activities of the recombinant protein, melanoma t-PA and trypsin were first determined in the S-2288 assay, and then the ability of comparable amounts of each to convert plasminogen was determined. Fig. 2B reveals that the ability of the recombinant protein to act upon the physiologic substrate is very similar to that of native t-PA. Although a nonspecific serine protease such as trypsin is able to convert plasminogen to plasmin, it does so much less efficiently than does either the native or recombinant plasminogen activator.

Both the purification scheme and the assays used to following purification required an intact and functional antigen combining site. In order to more quantitatively compare the recombinant molecule with antibody 59D8, we employed a simple competition assay. This assay measured the ability of soluble fibrin monomer to compete for antibody binding sites against fibrin bound to the bottom of a 96-well plate. Although the assay indicates that the native antibody binds fibrin monomer better than does the recombinant protein, the difference in their binding affinities is less than 10-fold (Fig. 3). It is evident that antibody binding is not significantly impaired in the fusion protein.

### DISCUSSION

Extensive analysis of the secreted protein indicates that a 59D8 heavy chain-t-PA fusion protein is being expressed and secreted in association with light chain in the manner predicted. The amount of recombinant protein present in cell culture supernatants, however, appears to be only 10 percent of that expected for monoclonal antibodies. By affinity purification, we routinely obtained only 0.1 µg of purified protein per milliliter of cell culture supernatant or 10 µg per ml in ascites. We monitored the purification with solid-phase immunoassays as described above, and our recoveries from the affinity columns were within the expected range. There are a number of possible reasons for the limited production of recombinant protein. One is that the recombinant protein is being degraded during cell growth or protein purification. In an attempt to limit proteolytic degradation, we have added protease inhibitors to the cell cultures. Although no improvement in yield was observed, proteolytic degradation remains a concern.

Other more fundamental problems could be the cause of the low yields of protein. Although messenger RNA of the appropriate size can be seen on Northern blot, transcription of the construct may occur at a low level. Transcription is driven by the natural heavy chain promoter and enhancer, but 3' sequences, which have been shown to be important in regulation of immunoglobulin expression, have been excluded from this construct. Gregor et al., Mol. Cell. Biol., 6:1903-1916 (1986); Kobrin et al., Mol. Cell. Biol., 6:1687-1697 (1986). In addition, the 3' untranslated region of the chimeric gene is from t-PA, a protein that is produced at a low level under normal conditions, and is subsequently stored in the cells where it is produced. It is possible that the 3' UT region of the t-PA gene leads to low levels of transcription or translation, or interferes with secretion of the recombinant protein from the cell. Experiments aimed at quantitation of mRNA synthesis, protein synthesis, and stability of the recombinant peptide should allow resolution of this problem.

Heavy chain loss variants provide a convenient tool for the reconstitution of the antibody combining site. Their availability makes it unnecessary to clone and transfect the productivity light chain rearrangement. This approach, of course, depends on being able to transfect these variant cell lines. The two lines used in these experiments were easily transfected using standard techniques, but it is not yet clear whether other SP2/0-derived lines will behave similarly. The amount of light chain that heavy chain loss variants secrete varies. However, some loss variants that secrete small quantities of light chain may be capable of secreting normal amounts of this same light chain when heavy chain synthesis is resumed. Wilde et al., Eur. J. Immunol., 10:462-467 (1980). Little is known about the biological basis for loss of immunoglobulin chain production in these cells and it is possible that the ability of some loss variants to produce light chain as well as heavy chain may be impaired. Our recombinant protein's low level of production could be the result of depressed light chain expression.

The recombinant t-PA β chain has a high level of catalytic activity, and it retains the specific ability to convert plasminogen to plasmin. Earlier studies, which linked staphylococcal nuclease and E. coli DNA polymerase functions to immunoglobulin heavy chain, yielded considerably less effector function activity than the 70 percent measured in the S-2288 assay. Neuberger et al., Nature, 312:604-608 (1984); Williams et al., Gene, 43:319-324 (1986). This retention of enzymatic activity and substrate specificity indicate that even complex molecules requiring strict folding and formation of multiple intrachain disulfide bonds can be used to form hybrid recombinant proteins. Others have shown that the β chain of t-PA is capable of folding correctly and maintaining activity in the absence of the α chain. MacDonald et al., Gene, 42:59-67 (1986); Von Zonneveld et al., Proc. Nat'l Acad. Sci. USA, 83:4670-4674 (1986). Our results confirm the activity of the catalytic chain alone, and indicate that the chain can fold correctly in the context of a different amino terminal sequence. Together, these observations provide evidence for the independent folding of different protein domains.

In summary, we have cloned the heavy chain gene coding for the antigen combining site of an antifibrin antibody and produced a construct that codes for a truncated heavy chain-t-PA β subunit fusion peptide. The construct was subsequently transfected into heavy chain loss variants of the antifibrin hybridoma. Western blot analysis indicates that the fusion protein has antifibrin antibody activity and retains a level of plasminogen activating activity high enough to be considered similar to that of native t-PA.

## Claims (Claims for the following Contracting State(s): CH DE FR GB IT LI LU NL SE)

1. A recombinant hybrid immunoglobulin molecule comprising an antibody, or a portion thereof, specific for fibrin and a plasminogen activator, or a portion thereof, capable of converting plasminogen to plasmin.

2. A molecule as claimed in claim 1, wherein the plasminogen activator is tissue-type plasminogen activator, streptokinase, urokinase or prourokinase.

3. A molecule as claimed in claim 1 or 2 which is antibody 64c5 (ATCC HB 8545) or 59D8 (ATCC HB 8546).

4. A molecule as claimed in any of the preceding claims wherein the portion of the plasminogen activator is the catalytic region.

5. A molecule as claimed in claim 4 wherein the catalytic region is the B chain of tPA or urokinase.

6. DNA coding for a recombinant hybrid immunoglobulin molecule as claimed in any of the preceding claims.

7. A pharmaceutical or in vivo diagnostically administrable composition comprising a recombinant hybrid immunoglobulin molecule as claimed in any of claims 1 to 5 and a pharmaceutically or veterinarily acceptable carrier therefor.

8. The use of a recombinant hybrid immunoglobulin molecule as claimed in any one of claims 1 to 5 in the preparation of a thrombolytic agent.

9. A recombinant hybrid immunoglobulin molecule as claimed in any of claims 1 to 5, which is detectably labelled.

10. A recombinant hybrid immunoglobulin molecule as claimed in claim 9, which is radiolabelled.

11. The use of a labelled recombinant hybrid immunoglobulin molecule as claimed in claim 9 or 10 in the preparation of a thrombus-detecting agent.

12. A process for the production of a recombinant hybrid immunoglobulin molecule according to any of claims 1 to 5, the process comprising coupling successive amino acids by peptide bond formation.

## Claims (Claims for the following Contracting State(s): ES GR)

1. A process for the production of a recombinant hybrid immunoglobulin molecule comprising an antibody, or a portion thereof, specific for fibrin and a plasminogen activator, or a portion thereof, capable of converting plasminogen to plasmin, the process comprising coupling successive amino acids by peptide bond formation.

2. A process as claimed in claim 1, wherein the plasminogen activator is tissue-type plasminogen activator, streptokinase, urokinase or prourokinase.

3. A process as claimed in claim 1 or 2 for preparing the antibody 64c5 (ATCC HB 8545) or 59D8 (ATCC HB 8546).

4. A process as claimed in any preceding claim wherein the portion of the plasminogen activator is the catalytic region.

5. A process as claimed in claim 4 wherein the catalytic region is the B chain of tPA or urokinase.

6. A process as claimed in any of claims 1 to 5, which additionally comprises detectably labelling the molecule.

7. A process as claimed in claim 6, which comprises radiolabelling the molecule.

8. A process for the preparation of a pharmaceutical or in vivo diagnostically administrable composition, the process comprising admixing a recombinant hybrid immunoglobulin molecule prepared by a process according to any of claims 1 to 5 with a pharmaceutically or veterinarily acceptable carrier therefor.

9. The use of a recombinant hybrid immunoglobulin molecule prepared by a process according to any of claims 1 to 5 in the preparation of a thrombolytic agent.

10. The use of a detectably labelled recombinant hybrid immunoglobulin molecule prepared by a process according to claim 6 or 7 in the preparation of a thrombus-detecting agent.

## Patentansprüche (Patentansprüche für folgende(n) Vertragsstaat(en): AT BE CH DE FR GB IT LI LU NL SE)

1. Rekombinantes Hybrid-Immunoglobulinmolekül, bestehend aus einem Antikörper oder einem Teil davon, der für Fibrin spezifisch ist, und einem Plasminogenaktivator oder einem Teil davon, der zur Konvertierung von Plasminogen zu Plasmin imstande ist.

2. Molekül nach Anspruch 1, wobei der Plasminogenaktivator ein gewebeartiger Plasminogenaktivator, Streptokinase, Urokinase oder Prourokinase ist.

3. Molekül nach Anspruch 1 oder 2, das Antikörper 64c5 (ATCC HB 8545) oder 59D8 (ATCC HB 8546) ist.

4. Molekül nach einem der vorangehenden Ansprüche, wobei der Teil des Plasminogenaktivators die katalytische Region ist.

5. Molekül nach Anspruch 4, wobei die katalytische Region die B-Kette von tPA oder Urokinase ist.

6. DNA, die für ein rekombinantes Hybrid-Immunoglobulinmolekül nach einem der vorangehenden Ansprüche kodiert.

7. Pharmazeutische oder in vivo diagnostisch verabreichbare Zusammensetzung, bestehend aus einem rekombinanten Hybrid-Immunoglobulinmolekül nach einem der Ansprüche 1 bis 5 und einem pharmazeutisch oder veterinärisch annehmbaren Träger dafür.

8. Verwendung eines rekombinanten Hybrid-Immunoglobulinmolekül nach einem der Ansprüche 1 bis 5 in der Herstellung eines Thrombolytikums.

9. Rekombinantes Hybrid-Immunoglobulinmolekül nach einem der Ansprüche 1 bis 5, das nachweisbar markiert ist.

10. Rekombinantes Hybrid-Immunoglobulinmolekül nach Anspruch 9, das radiomarkiert ist.

11. Verwendung eines markierten rekombinanten Hybrid-Immunoglobulinmoleküls nach Anspruch 9 oder 10 in der Herstellung eines Thrombuserkennungsmittels.

12. Verfahren zur Herstellung eines rekombinanten Hybrid-Immunoglobulinmoleküls nach einem der Ansprüche 1 bis 5, wobei das Verfahren die Kopplung aufeinanderfolgender Aminosäuren durch Bildung von Peptidbindungen umfaßt.

## Patentansprüche (Patentansprüche für folgende(n) Vertragsstaat(en): ES GR)

1. Verfahren zur Herstellung eines rekombinanten Hybrid-Immunoglobulinmoleküls, bestehend aus einem Antikörper oder einem Teil davon, der für Fibrin spezifisch ist, und einem Plasminogenaktivator oder einem Teil davon, der zur Konvertierung von Plasminogen zu Plasmin imstande ist, wobei das Verfahren die Kopplung aufeinanderfolgender Aminosäuren durch Bildung von Peptidbindungen umfaßt.

2. Verfahren nach Anspruch 1, wobei der Plasminogenaktivator ein gewebeartiger Plasminogenaktivator, Streptokinase, Urokinase oder Prourokinase ist.

3. Verfahren nach Anspruch 1 oder 2 zur Herstellung des Antikörpers 64c5 (ATCC HB 8545) oder 59D8 (ATCC HB 8546).

4. Verfahren nach einem der vorangehenden Ansprüche, wobei der Teil des Plasminogenaktivators die katalytische Region ist.

5. Verfahren nach Anspruch 4, wobei die katalytische Region die B-Kette von tPA oder Urokinase ist.

6. Verfahren nach einem der Ansprüche 1 bis 5, das zusätzlich das nachweisbare Markieren des Moleküls umfaßt.

7. Verfahren nach Anspruch 6, welches das Radiomarkieren des Moleküls umfaßt.

8. Verfahren zur Herstellung einer pharmazeutischen oder in vivo diagnostisch verabreichbaren Zusammensetzung, wobei das Verfahren das Beimengen eines rekombinanten Hybrid-Immunoglobulinmoleküls, das durch ein Verfahren nach einem der Ansprüche 1 bis 5 hergestellt wurde, zu einem pharmazeutisch oder veterinärisch annehmbaren Träger umfaßt.

9. Verwendung eines rekombinanten Hybrid-Immunoglobulinmoleküls, das durch ein Verfahren nach einem der Ansprüche 1 bis 5 hergestellt wurde, in der Herstellung eines Thrombolytikums.

10. Verwendung eines nachweisbar markierten rekombinanten Hybrid-Immunoglobulinmoleküls, das durch ein Verfahren nach einem der Ansprüche 6 oder 7 hergestellt wurde, in der Herstellung eines Thromboserkennungsmittels.

## Revendications (Revendications pour l'(les) Etat(s) contractant(s) suivant(s): AT BE CH DE FR GB IT LI LU NL SE)

1. Molécule d'immunoglobuline hybride recombinante comprenant un anticorps, ou une partie de celui-c,i spécifique pour la fibrine et un activateur du plasminogène, ou une partie de celui-ci, capable de convertir le plasminogène en plasmine.

2. Molécule suivant la revendication 1, dans laquelle l'activateur du plasminogène est l'activateur du plasminogène de type tissulaire, la streptokinase, l'urokinase ou la prourokinase.

3. Molécule suivant la revendication 1 ou 2, qui est l'anticorps 64c5 (ATCC HB 8545) ou 59D8 (ATCC HB 8546).

4. Molécule suivant l'une quelconque des revendications précédentes, dans laquelle la partie de l'activateur du plasminogène est la région catalytique.

5. Molécule suivant la revendication 4, dans laquelle la région catalytique est la chaîne B de tPA ou l'urokinase.

6. ADN codant pour une molécule d'immunoglobuline hybride recombinante suivant l'une quelconque des revendications précédentes.

7. Composition pharmaceutique ou à administrer à des fins de diagnostic in vivo, comprenant une molécule d'immunoglobuline hybride recombinante suivant l'une quelconque des revendications 1 à 5 et un excipient pharmaceutiquement ou vétérinairement acceptable.

8. Utilisation d'une molécule d'immunoglobuline hybride recombinante suivant l'une quelconque des revendications 1 à 5 dans la préparation d'un agent thrombolytique.

9. Molécule d'immunoglobuline hybride recombinante suivant l'une quelconque des revendications 1 à 5 , qui est marquée de façon détectable.

10. Molécule d'immunoglobuline hybride recombinante suivant la revendication 9, qui est radiomarquée.

11. Utilisation d'une molécule d'immunoglobuline hybride recombinante marquée suivant la revendication 9 ou 10 dans la préparation d'un agent de détection des thrombus.

12. Procédé de production d'une molécule d'immunoglobuline hybride recombinante suivant l'une quelconque des revendications 1 à 5, le procédé comprenant la condensation d'acides aminés successifs par formation de liaisons peptidiques.

## Revendications (Revendications pour l'(les) Etat(s) contractant(s) suivant(s): ES GR)

1. Procédé de production d'une molécule d'immunoglobuline hybride recombinante comprenant un anticorps, ou une partie de celui-ci, spécifique pour la fibrine et un activateur du plasminogène, ou une partie de celui-ci, capable de convertir le plasminogène en plasmine, le procédé comprenant la condensation d'acides aminés successifs par formation de liaisons peptidiques.

2. Procédé suivant la revendication 1, dans lequel l'activateur du plasminogène est l'activateur du plasminogène de type tissulaire, la streptokinase, l'urokinase ou la prourokinase.

3. Procédé suivant la revendication 1 ou 2 de préparation de l'anticorps 64c5 (ATCC HB 8545) ou 59D8 (ATCC HB 8546).

4. Procédé suivant l'une quelconque des revendications précédentes, dans lequel la partie de l'activateur du plasminogène est la région catalytique.

5. Procédé suivant la revendication 4, dans lequel la région catalytique est la chaîne B de tPA ou l'urokinase.

6. Procédé suivant l'une quelconque des revendications 1 à 5, qui comprend de plus le marquage de la molécule de façon détectable.

7. Procédé suivant la revendication 6, qui comprend le radiomarquage de la molécule.

8. Procédé de préparation d'une composition pharmaceutique ou à administrer à des fins de diagnostic in vivo, le procédé comprenant le mélange d'une molécule d'immunoglobuline hybride recombinante préparée par un procédé suivant l'une quelconque des revendications 1 à 5 avec un excipient pharmaceutiquement ou vétérinairement acceptable.

9. Utilisation d'une molécule d'immunoglobuline hybride recombinante préparée par un procédé suivant l'une quelconque des revendications 1 à 5 dans la préparation d'un agent thrombolytique.

10. Utilisation d'une molécule d'immunoglobuline hybride recombinante marquée de façon détectable préparée par un procédé suivant la revendication 6 ou 7 dans la préparation d'un agent de détection des thrombus.
